# EUROPÄISCHE PATENTANMELDUNG

(11) **EP 3 136 028 A1**
(43) Veröffentlichungstag der Anmeldung: **01.03.2017**
(21) Anmeldenummer: 15182895.1
(22) Anmeldetag: 28.08.2015
(51) Int. Cl.: F25J 3/02, C10G 70/04, C07C 7/11

(54) **VERFAHREN UND ANLAGE ZUR TRENNTECHNISCHEN BEARBEITUNG EINES AUSGANGSGEMISCHS**

(71) Anmelder: Linde Aktiengesellschaft, 80331 München (DE)
(72) Erfinder: Pham Duc, Tuat, 82377 Penzberg (DE); Kuhn, Paul, 82065 Baierbrunn (DE)
(74) Vertreter: Frommberger, Moritz

(57) **Zusammenfassung**

Die Erfindung betrifft ein Verfahren (100) zur trenntechnischen Bearbeitung eines Ausgangsgemischs (a), das überwiegend Wasserstoff, Methan und Kohlenwasserstoffe mit zwei Kohlenstoffatomen enthält, wobei das Verfahren (100) ein Bilden eines ersten Trenneinsatzes unter Verwendung des Ausgangsgemischs und ein absorptives Trennen (12) des ersten Trenneinsatzes unter Verwendung einer Absorptionsflüssigkeit (c), die überwiegend Kohlenwasserstoffe mit zumindest drei Kohlenstoffatomen enthält umfasst, wobei das absorptive Trennen (c) des ersten Trenneinsatzes das Erzeugen eines ersten Trennprodukts (b), das überwiegend Wasserstoff und Methan aus dem ersten Trenneinsatz enthält, und eines zweiten Trennprodukts (d), das Wasserstoff, Methan und Kohlenwasserstoffe mit zwei Kohlenstoffatomen aus dem ersten Trenneinsatz und Kohlenwasserstoffe mit zumindest drei Kohlenstoffatomen aus der Absorptionsflüssigkeit enthält, umfasst. Es ist das Bilden eines zweiten Trenneinsatzes unter Verwendung des zweiten Trennprodukts (d) und ein destillatives Trennen (2) des zweiten Trenneinsatzes vorgesehen, wobei das destillative Trennen (2) des zweiten Trenneinsatzes das Erzeugen eines dritten Trennprodukts (p), das überwiegend Methan, Wasserstoff und Kohlenwasserstoffe mit zwei Kohlenstoffatomen enthält, und das Erzeugen eines vierten Trennprodukts (f), das überwiegend Kohlenwasserstoffe mit zumindest drei Kohlenstoffatomen enthält, umfasst. Ferner ist das Bilden eines dritten Trenneinsatzes unter Verwendung des dritten Trennprodukts (p) und ein destillatives Trennen (6) des dritten Trenneinsatzes vorgesehen, wobei das destillative Trennen (6) des dritten Trenneinsatzes das Erzeugen eines fünften Trennprodukts (s), das überwiegend Methan, Wasserstoff und Kohlenwasserstoffe mit zwei Kohlenstoffatomen enthält, und das Erzeugen eines sechsten Trennprodukts (g), das überwiegend Kohlenwasserstoffe mit zwei Kohlenstoffatomen enthält, umfasst. Schließlich ist ein Verwenden des fünften Trennprodukts (s) beim Bilden des ersten Trenneinsatzes vorgesehen. Eine entsprechende Anlage ist ebenfalls Gegenstand der Erfindung.

## Beschreibung

Die Erfindung betrifft ein Verfahren zur trenntechnischen Bearbeitung eines überwiegend Wasserstoff, Methan und Kohlenwasserstoffe mit zwei Kohlenstoffatomen enthaltenden Ausgangsgemischs und eine entsprechende Anlage gemäß den Oberbegriffen der unabhängigen Patentansprüche.

### Stand der Technik

Verfahren und Anlagen zum Dampfspalten von Kohlenwasserstoffen sind beispielsweise im Artikel "Ethylene" in Ullmann's Encyclopedia of Industrial Chemistry, Onlineausgabe, 15. April 2007, DOI 10.1002/14356007.a10_045.pub2, beschrieben. Das Dampfspalten (engl. Steam Cracking) wird beispielsweise zur Gewinnung von kurzkettigen Olefinen wie Ethylen und Propylen, Diolefinen wie Butadien oder von Aromaten eingesetzt, ist jedoch nicht hierauf beschränkt.

Durch Dampfspalten wird dabei ein Stoffgemisch erhalten, das zunächst als Rohgas bezeichnet wird. Dieses wird mehreren Aufbereitungsschritten, beispielsweise einer Rohgaswäsche, einer Rohgasverdichtung und einer sogenannten Primärfraktionierung unterworfen. Das auf diese Weise aufbereitete Rohgas wird anschließend einer Trennung zugeführt, die zur Gewinnung von Komponenten oder Komponentengruppen des Rohgases dient. Es kann auch vorgesehen sein, bestimmte Komponenten des Rohgases vor oder innerhalb einer entsprechenden Trennung, beispielsweise durch Hydrieren, umzusetzen.

Eine typische Trennung umfasst mehrere Trennschritte, bei denen jeweils Komponentengruppen gewonnen werden. Beispielsweise sind hierbei "Demethanizer First"-, "Deethanizer First"- oder auch "Depropanizer First"- Verfahren bekannt. Zu Details sei auf die Fachliteratur, beispielsweise den erwähnten Artikel "Ethylene" in Ullmann's Encyclopedia of Industrial Chemistry, verwiesen.

Die vorliegende Erfindung betrifft dabei insbesondere Trennungen, in denen ein überwiegend Wasserstoff, Methan und Kohlenwasserstoffe mit zwei Kohlenstoffatomen enthaltendes Stoffgemisch, ein sogenannter C2minus-Strom (siehe unten) gebildet wird, aus welchem die Kohlenwasserstoffe mit zwei Kohlenstoffatomen zunächst in einer gemeinsamen Fraktion gewonnen werden sollen. Ein derartiges, überwiegend Wasserstoff, Methan und Kohlenwasserstoffen mit zwei Kohlenstoffatomen enthaltendes bzw. an diesen Komponenten reiches Stoffgemisch wird nachfolgend auch als "Ausgangsgemisch" bezeichnet.

Zur trenntechnischen Behandlung entsprechender Ausgangsgemische sind beispielsweise die unten erläuterten ausschließlich oder überwiegend destillativen Verfahren aus dem Stand der Technik bekannt, die je nach den beim Dampfspalten umgesetzten Verbindungen unterschiedlich ausgestaltet sein können. Diesen Verfahren ist gemeinsam, dass hierbei Temperaturen von -100 °C und weniger zur Abtrennung von Methan und Wasserstoff zum Einsatz kommen müssen, was die Verwendung von Ethylen als Kältemittel erfordert. Gegebenenfalls ist dabei der Einsatz von Turboexpandern zur Erzielung von Temperaturen von -130 °C und weniger erforderlich, insbesondere dann, wenn beim Dampfspalten überwiegend oder ausschließlich gasförmige bzw. ethanreiche Einsätze verwendet werden. Dies erweist sich aufgrund der hierbei erforderlichen Materialien und Apparate als aufwendig und ausgesprochen kostenintensiv.

Zur Vermeidung von entsprechend niedrigen Temperaturen sind auch teilweise absorptive Verfahren bekannt. Beispielsweise wird in der EP 0 675 094 A2 vorgeschlagen, ein entsprechendes Ausgangsgemisch in einen Absorber einzuspeisen und die Kohlenwasserstoffe mit zwei Kohlenstoffatomen mittels einer Absorptionsflüssigkeit unter Verbleib eines überwiegend oder ausschließlich Methan und Wasserstoff enthaltenden Gasgemischs auszuwaschen. Hierbei gehen neben den Kohlenwasserstoffen mit zwei Kohlenstoffatomen auch Anteile des Methans und des Wasserstoffs aus dem Ausgangsgemisch in die Absorptionsflüssigkeit über. Methan und Wasserstoff werden daher zunächst aus dem beladenen Absorptionsflüssigkeit ausgetrieben, ehe aus diesem die Kohlenwasserstoffe mit zwei Kohlenstoffatomen zurückgewonnen werden. Weitere teilweise absorptive Verfahren in unterschiedlicher Ausgestaltung sind beispielsweise auch aus der US 2 815 650 A, der US 5 710 357 A, der WO 2014/064172 A2 und der US 2 933 901 A1 bekannt.

Die Erfindung stellt sich die Aufgabe, entsprechende Verfahren und Anlagen zu vereinfachen und ihren Betrieb effizienter zu gestalten.

### Offenbarung der Erfindung

Diese Aufgabe wird durch ein Verfahren zur trenntechnischen Bearbeitung eines überwiegend Wasserstoff, Methan und Kohlenwasserstoffe mit zwei Kohlenstoffatomen enthaltenden Ausgangsgemischs und eine entsprechende Anlage mit den Merkmalen der unabhängigen Patentansprüche gelöst. Ausgestaltungen sind jeweils Gegenstand der abhängigen Patentansprüche und der nachfolgenden Beschreibung.

Vor der Erläuterung der Merkmale und Vorteile der vorliegenden Erfindung werden deren Grundlagen und die verwendeten Begriffe erläutert.

Gängige Verfahren zur Trennung von Produktströmen aus Verfahren zur Herstellung von Kohlenwasserstoffen wie des eingangs erwähnten Spaltgases umfassen die Bildung einer Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (und konventionsgemäß unter Umständen auch Wasserstoff, dann auch "C1 minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt für die "C5-Fraktion" und die höheren Fraktionen. Mehrere solcher Fraktionen können zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen sowie ggf. Wasserstoff und Methan. Entsprechende Fraktionen können auch als Kältemittel eingesetzt werden, so beispielsweise die C2- oder C3-Fraktionen. Die durch entsprechende C2- oder C3-Kältemittel bereitstellbaren Temperaturniveaus werden landläufig auch als "C2-Kälte" oder "C3-Kälte" bezeichnet. Diese Kältemittel werden in Kältekreisläufen geführt, wo sie zuerst auf ein bestimmtes Enddruckniveau verdichtet und ausgehend von diesem anschließend auf unterschiedliche Druckniveaus zur Kälterzeugung bei entsprechenden Temperaturniveaus entspannt werden. Mittels eines C3-Kältemittels (insbesondere Propan und Propylen) lassen sich auf diese Weise beispielsweise Kältemittelströme mit ca. -40 °C und +10 °C erzeugen.

Flüssige und gasförmige Ströme bzw. flüssige und gasförmige Gemische können im hier verwendeten Sprachgebrauch reich oder arm an einer oder mehreren Komponenten sein, wobei "reich" für einen Gehalt von wenigstens 90%, 95%, 99%, 99,5%, 99,9%, 99,99% oder 99,999% und "arm" für einen Gehalt von höchstens 10%, 5%, 1 %, 0,1%, 0,01% oder 0,001% auf molarer, Gewichts- oder Volumenbasis stehen kann. Flüssige und gasförmige Ströme bzw. flüssige und gasförmige Gemische können im hier verwendeten Sprachgebrauch ferner angereichert oder abgereichert an einer oder mehreren Komponenten sein, wobei sich diese Begriffe auf einen entsprechenden Gehalt in einem Ursprungsgemisch beziehen, aus dem der flüssige oder gasförmige Strom bzw. das flüssige oder gasförmige Gemisch jeweils erhalten wurde. Der flüssige oder gasförmige Strom bzw. das flüssige oder gasförmige Gemisch ist "angereichert", wenn dieser bzw. dieses zumindest den 1,1-fachen, 1,5-fachen, 2-fachen, 5-fachen, 10-fachen, 100-fachen oder 1.000-fachen Gehalt, "abgereichert", wenn er bzw. es höchstens den 0,9-fachen, 0,5-fachen, 0,1-fachen, 0,01-fachen oder 0,001-fachen Gehalt einer entsprechenden Komponente, bezogen auf das Ursprungsgemisch, enthält. Ein "überwiegend" eine oder mehrere Komponenten enthaltender Strom bzw. ein entsprechendes Gemisch enthält diese eine oder mehreren Komponenten zumindest zu 80% oder ist reich an dieser bzw. diesen im obigen Sinn.

Ist nachfolgend davon die Rede, dass ein Gemisch, das einem Trennverfahren unterworfen wird, also ein "Trenneinsatz", unter Verwendung eines Ursprungsgemischs, das beispielsweise in einem anderen Verfahren erzeugt oder auf andere Weise bereitgestellt wird, "gebildet" wird, bedeutet dies, dass dieses zumindest einige in dem Ursprungsgemisch enthaltene oder aus diesem erhaltene Komponenten aufweist. Ein in diesem Sinne gebildeter Trenneinsatz kann aus dem Ursprungsgemisch durch Abtrennen oder Abzweigen eines Teilstroms oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten werden. Ein Trenneinsatz kann auch unter Verwendung des gesamten Ursprungsgemischs und ggf. weiterer Komponenten gebildet werden. Insbesondere umfasst das "Bilden" eines Trenneinsatzes auch lediglich das Überführen des Ursprungsgemischs und ggf. weiterer Komponenten mittels einer Leitung in eine entsprechende Trenneinrichtung.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Zur Auslegung und spezifischen Ausgestaltung von Destillationssäulen und Absorptionskolonnen, wie sie auch im Rahmen der vorliegenden Anmeldung eingesetzt werden können, sei auf einschlägige Lehrbücher verwiesen (siehe beispielsweise Sattler, K.: Thermische Trennverfahren: Grundlagen, Auslegung, Apparate, 3. Auflage 2001, Weinheim, Wiley-VCH).

Bei einer "Destillationssäule" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, bereitgestelltes Stoffgemisch (Trenneinsatz) zumindest teilweise aufzutrennen, also aus dem Stoffgemisch jeweils Reinstoffe oder Stoffgemische zu erzeugen, die gegenüber dem Stoffgemisch bezüglich zumindest einer Komponente angereichert bzw. abgereichert im oben erläuterten Sinne sind. Destillationssäulen sind aus dem Bereich der Trenntechnik hinlänglich bekannt. Typischerweise sind Destillationssäulen als zylindrische Metallbehälter ausgebildet, die mit Einbauten, beispielsweise Siebböden oder geordneten oder ungeordneten Packungen, ausgerüstet sind. Eine Destillationssäule zeichnet sich unter anderem dadurch aus, dass sich in ihrem unteren Bereich, auch als Sumpf bezeichnet, eine flüssige Fraktion abscheidet. Diese flüssige Fraktion, auch als Sumpfprodukt bezeichnet, wird in einer Destillationssäule mittels eines Sumpfverdampfers erwärmt, so dass kontinuierlich ein Teil des Sumpfprodukts verdampft und in der Destillationssäule gasförmig aufsteigt. Eine Destillationssäule ist ferner typischerweise mit einem sogenannten Kopfkondensator versehen, in den zumindest ein Teil eines sich in einem oberen Bereich der Destillationssäule anreichernden Gasgemischs oder ein entsprechendes Reingas, auch als Kopfprodukt bezeichnet, eingespeist, dort verflüssigt und als flüssiger Rücklauf am Kopf der Destillationssäule aufgegeben wird.

Im Gegensatz zu einer Destillationssäule verfügt eine "Absorptionskolonne" nicht über einen Sumpfverdampfer. Auch Absorptionskolonnen sind aus dem Bereich der Trenntechnik allgemein bekannt. Absorptionskolonnen werden zur Absorption im Phasengegenstrom verwendet und daher auch als Gegenstromkolonnen bezeichnet. Bei der Absorption im Gegenstrom strömt die abgebende Gasphase aufwärts durch eine Absorptionskolonne. Die aufnehmende Lösungsphase fließt, von oben aufgegeben und unten abgezogen, der Gasphase entgegen. In einer entsprechenden Absorptionskolonne sind ebenfalls typischerweise Einbauten vorgesehen, die für einen stufenweisen (Böden, Sprühzonen, rotierende Teller usw.) oder stetigen (regellose Schüttungen von Füllkörpern, Packungen usw.) Phasenkontakt sorgen.

Bei einer Absorption im Gegenstrom, wie er in einer Absorptionskolonne erfolgt, wird ein gasförmiger Strom einem insbesondere fein verteilten flüssigen Strom entgegengeschickt. Der flüssige Strom nimmt dabei Komponenten aus dem gasförmigen Strom auf, die auf diese Weise aus dem gasförmigen Strom ausgewaschen werden. Die Auswaschung kann teilweise oder vollständig sein.

### Vorteile der Erfindung

Die vorliegende Erfindung geht von einem Verfahren zur trenntechnischen Bearbeitung eines überwiegend Wasserstoff, Methan und Kohlenwasserstoffe mit zwei Kohlenstoffatomen enthaltenden Ausgangsgemischs, also eines typischen C2minus-Stroms, aus. Das Verfahren umfasst, unter Verwendung des Ausgangsgemischs einen ersten Trenneinsatz zu bilden und diesen absorptiv zu trennen, also beispielsweise das Ausgangsgemisch vollständig oder teilweise, und im vorliegenden Fall zusammen mit einem unten erläuterten weiteren Stoffgemisch, in eine Absorptionskolonne einzuspeisen.

Bei der absorptiven Trennung wird im Rahmen der vorliegenden Erfindung eine Absorptionsflüssigkeit eingesetzt, die überwiegend Kohlenwasserstoffe mit zumindest drei Kohlenstoffatomen enthält. Bei dem absorptiven Trennen des ersten Trenneinsatzes wird ein erstes Trennprodukt, das überwiegend Wasserstoff und Methan aus dem ersten Trenneinsatz enthält, erzeugt. Außerdem wird hierbei ein zweites Trennprodukt, das Wasserstoff, Methan und Kohlenwasserstoffe mit zwei Kohlenstoffatomen aus dem ersten Trenneinsatz und Kohlenwasserstoffe mit zumindest drei Kohlenstoffatomen aus der Absorptionsflüssigkeit enthält, erzeugt. Das erste Trennprodukt, das überwiegend Wasserstoff und Methan aus dem ersten Trenneinsatz enthält, wird typischerweise einem anschließenden Trennschritt unterworfen, um Wasserstoff und Brenngas zu gewinnen, wie unten erläutert. Das zweite Trennprodukt wird im Rahmen der vorliegenden Erfindung wie nachfolgend erläutert weiter behandelt. Ist daher hier von "Trennprodukten" die Rede, seien darunter Produkte des jeweiligen Trennschritts, nicht Produkte des Verfahrens insgesamt, verstanden.

Die vorliegende Erfindung sieht nun vor, unter Verwendung des zweiten Trennprodukts, also jenes Trennprodukts der absorptiven Trennung, das Wasserstoff, Methan und Kohlenwasserstoffe mit zwei Kohlenstoffatomen aus dem ersten Trenneinsatz und Kohlenwasserstoffe mit zumindest drei Kohlenstoffatomen aus der Absorptionsflüssigkeit enthält, einen zweiten Trenneinsatz zu bilden und diesen destillativ zu trennen. Das destillative Trennen des zweiten Trenneinsatzes umfasst dabei das Erzeugen eines dritten Trennprodukts, das überwiegend Methan, Wasserstoff und Kohlenwasserstoffe mit zwei Kohlenstoffatomen enthält, und das Erzeugen eines vierten Trennprodukts, das überwiegend Kohlenwasserstoffe mit zumindest drei Kohlenstoffatomen enthält. Der Wasserstoff, das Methan und die Kohlenwasserstoffe mit zwei Kohlenstoffatomen in dem dritten Trennprodukt stammen dabei ursprünglich aus dem ersten Trenneinsatz, die Kohlenwasserstoffe mit zumindest drei Kohlenstoffatomen in dem vierten Trennprodukt aus der Absorptionsflüssigkeit, die bei der absorptiven Trennung eingesetzt wird. Bei dem vierten Trennprodukt handelt es sich damit um die regenerierte und von absorbierten Komponenten befreite Absorptionsflüssigkeit, die auf diese Weise erneut für die absorptive Trennung des ersten Trenneinsatzes eingesetzt werden kann. Das dritte Trennprodukt enthält noch Wasserstoff und Methan, weil dieses zuvor nicht aus dem zweiten Trennprodukt entfernt wurde und bei der absorptiven Trennung aus dem ersten Trenneinsatz teilweise in das zweite Trennprodukt übergegangen ist.

Im Rahmen der vorliegenden Erfindung ist daher vorgesehen, unter Verwendung des dritten Trennprodukts einen dritten Trenneinsatz zu bilden und auch diesen destillativ zu trennen, wobei das destillative Trennen des dritten Trenneinsatzes das Erzeugen eines fünften Trennprodukts, das überwiegend Methan, Wasserstoff und Kohlenwasserstoffe mit zwei Kohlenstoffatomen enthält, und das Erzeugen eines sechsten Trennprodukts, das überwiegend Kohlenwasserstoffe mit zwei Kohlenstoffatomen enthält, umfasst. Das Trennen des dritten Trenneinsatzes erfolgt dabei in einem auch als "Soft Demethanizer" bezeichenbaren Trennschritt bzw. einer entsprechenden Vorrichtung, also einer Vorrichtung, die nicht scharf zwischen Methan und Wasserstoff am Kopf und Kohlenwasserstoffen mit zwei Kohlenwasserstoffen im Sumpf trennt. Zwar enthält auf diese Weise das Kopfprodukt (im Sprachgebrauch dieser Anmeldung das "fünfte Trennprodukt") noch Anteile an Kohlenwasserstoffen mit zwei Kohlenstoffatomen, das Verfahren bietet jedoch in dieser Ausgestaltung den Vorteil, dass keine tiefen Temperaturen, wie sie nur mit C2-Kältemitteln bereitgestellt werden könnten, erforderlich sind. Im vorliegenden Fall kann das destillative Trennen des dritten Trenneinsatzes beispielsweise mittels einer Destillationseinheit erfolgen, die einen mit Kältemittel auf ca. -38 °C betriebenen Kopfkondensator aufweist. In herkömmlichen Demethanizern ist hingegen C2-Kälte erforderlich.

Im Rahmen der vorliegenden Erfindung ist die "unscharfe" Trennung des dritten Trenneinsatzes nicht von Nachteil, weil hier das fünfte Trennprodukt beim Bilden des ersten Trenneinsatzes verwendet wird. Das fünfte Trennprodukt oder ein Teil davon wird also der absorptiven Trennung zusammen mit dem Ausgangsgemisch oder einem Teil davon wieder zugeführt. Auf diese Weise können die in dem fünften Trennprodukt enthaltenen Kohlenwasserstoffe mit zwei Kohlenstoffatomen aus dem fünften Trennprodukt zurückgewonnen werden. Das Verfahren erlaubt damit insgesamt eine vollständige Abtrennung von Wasserstoff und Methan von den Kohlenwasserstoffen mit zwei Kohlenstoffatomen aus dem Ausgangsgemisch, ohne dass C2-Kältemittel eingesetzt werden müssten. Die Vorteile gegenüber dem in der EP 0 675 094 A2 vorgeschlagenen Verfahren bestehen darin, dass weniger Absorptionsflüssigkeit und somit ein geringerer Energieaufwand für die gleiche Trennaufgabe erforderlich ist. Darüber hinaus können die Trennsäulen und die Verfahrensführung einfacher gestaltet werden. Besondere Vorteile ergeben sich, wenn die absorptive Trennung und die Trennung des dritten Trenneinsatzes in einer integrierten Einheit ohne Zwischenaufkocher und Zwischenkühler ausgeführt werden, wie unten erläutert.

Besondere Vorteile hat ein erfindungsgemäßes Verfahren, wenn die absorptive Trennung des ersten Trenneinsatzes auf einem ersten Druckniveau und die destillative Trennung des zweiten Trenneinsatzes auf einem zweiten Druckniveau durchgeführt wird, wobei das zweite Druckniveau 1 bis 10 bar oberhalb des ersten Druckniveaus liegt. Das zweite Druckniveau kann insbesondere 1 bis 5 bar, beispielsweise 2 bis 4 oder 2 bis 3 bar, oberhalb des ersten Druckniveaus liegen. So kann das erste Druckniveau beispielsweise bei 25 bis 35 bar, insbesondere bei 28 bis 32 bar, vorzugsweise bei ca. 30 bar, und das zweite Druckniveau beispielsweise bei 30 bis 40 bar, insbesondere bei 32 bis 34 bar, vorzugsweise bei ca. 33 bar, liegen. Liegt das zweite Druckniveau oberhalb des ersten, ist es möglich, Gas aus der destillativen Trennung des zweiten Trenneinsatzes, insbesondere das dritte Trennprodukt, ohne Einsatz von Verdichtern in die nachgeschaltete destillative Trennung des dritten Trenneinsatzes zu führen, die vorzugsweise auf dem ersten Druckniveau durchgeführt wird, und das hier erhaltene fünfte Trennprodukt wieder als Teil des ersten Trenneinsatzes in die absorptive Trennung einzuspeisen. Ein entsprechendes Verfahren kommt daher vollständig ohne teure und wartungsanfällige Verdichter aus. Hierin besteht ein Vorteil beispielsweise gegenüber dem in der US 2 933 901 A1 vorgeschlagenen Verfahren.

Der Druckunterschied zwischen der absorptiven Trennung des ersten Trenneinsatzes und der destillativen Trennung des zweiten Trenneinsatzes kann durch die Verwendung einer Pumpe erzeugt werden, die das zweite Trennprodukt bzw. den unter Verwendung des zweiten Trennprodukts gebildeten zweiten Trenneinsatz vor der Einspeisung in die destillative Trennung flüssig auf Druck bringt.

An dieser Stelle sei erwähnt, dass das erste, das dritte und das fünfte Trennprodukt bei den jeweiligen Trennungen jeweils in gasförmigem Zustand, das zweite, das vierte und das sechste Trennprodukt hingegen jeweils in flüssiger Form vorliegen. Für die absorptive Trennung wird dabei insbesondere eine Absorptionskolonne bzw. eine entsprechende absorptiv arbeitende Trenneinheit eingesetzt, für die destillativen Trennungen hingegen Destillationssäulen bzw. entsprechende Trenneinheiten. Das erste, das dritte und das fünfte Trennprodukt werden den genannten Trenneinheiten dabei am Kopf, das zweite, vierte und sechste Trennprodukt werden den genannten Trenneinheiten hingegen aus dem Sumpf entnommen.

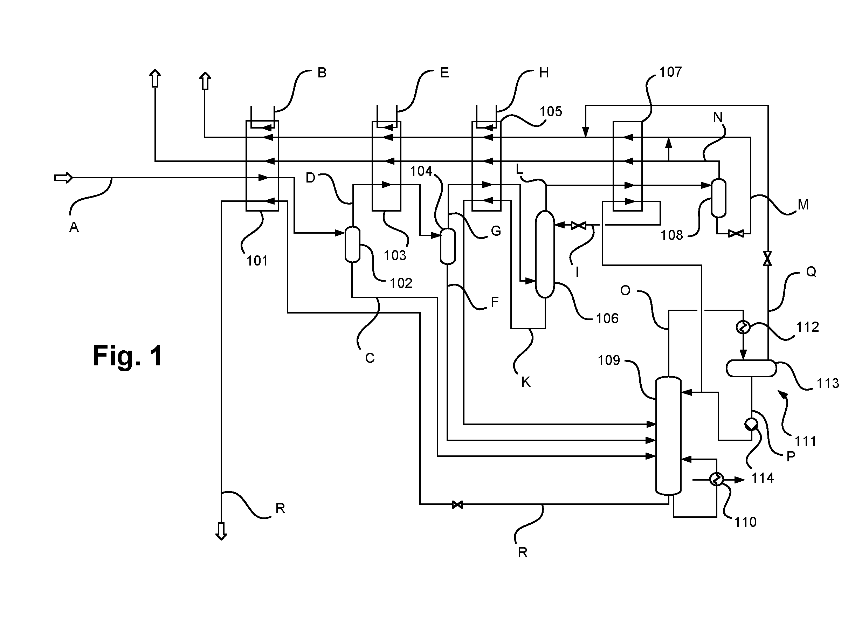

Im Rahmen der vorliegenden Erfindung sind insbesondere die vergleichsweise hohen, bei den Trennungen eingesetzten Temperaturen von Vorteil. So ist es insbesondere von Vorteil, wenn das absorptive Trennen des ersten Trenneinsatzes auf einem Temperaturniveau von -40 bis -25 °C, insbesondere von -37 °C bis -27 °C, vorzugsweise bei ca. -32 °C, durchgeführt wird. Wird das Ausgangsgemisch bzw. der erste Trenneinsatz beispielsweise einem dem erfindungsgemäßen Verfahren vorgelagerten C3-Absorber-/Deethanizersystem (in diesem Fall liegt das Temperaturniveau typischerweise bei ca. -37 °C) oder auch einer Front-End-C2-Hydrierung (in diesem Fall liegt das Temperaturniveau typischerweise bei ca. -37 °C) entnommen, sind zur Erreichung eines entsprechenden Temperaturniveaus keine zusätzlichen Vorkühlschritte erforderlich, so dass sich weitere Apparate und C3-Kältemittel einsparen lassen bzw. Kältekreisläufe kleiner dimensioniert werden können. Auch die bei der absorptiven Trennung eingesetzte Absorptionsflüssigkeit kann auf einem derartigen, vergleichsweise hohen Temperaturniveau verwendet werden. Dies stellt einen Vorteil gegenüber einem herkömmlichen "C2-Absorber" dar, wie er beispielsweise in der unten erläuterten Figur 1 gezeigt ist.

Auch für die destillative Trennung des zweiten Trenneinsatzes sind im Rahmen der vorliegenden Erfindung keine ausgesprochen tiefen Temperaturen erforderlich. So kann beispielsweise ein Kopfkondensator für eine Destillationssäule, die für die destillative Trennung des zweiten Trenneinsatzes verwendet wird, mit Kältemittel auf einem Temperaturniveau von -25 bis -15 °C, insbesondere von -22 bis -18 °C, vorzugsweise ca. -20 °C, gekühlt werden. Bei einer derartigen Trennung kommt also ein Kältemittel auf einem entsprechenden Temperaturniveau zum Einsatz und das dabei gebildete dritte Trennprodukt wird auf einem entsprechenden Temperaturniveau bzw. einem Temperaturniveau, das geringfügig höher liegt, bereitgestellt. Es kann beispielsweise Mitteldruck-C3-Kältemittel eingesetzt werden, wie es in den verwendeten Kältekreisläufen vorhanden ist.

Wie erwähnt, reicht in dem verwendeten "Soft Demethanizer", d.h. in der zur destillativen Trennung des dritten Trenneinsatzes verwendeten Destillationssäule, eine unscharfe Trennung aus. Es sind daher auch hier vergleichsweise hohe Temperaturen ausreichend, so dass ein Kopfkondensator einer zur destillativen Trennung des dritten Trenneinsatzes verwendeten Destillationssäule beispielsweise mit Kältemittel auf einem Temperaturniveau von -45 bis -35 °C, insbesondere -36 bis -40 °C, vorzugsweise ca. -38 °C, d.h. C3-Kältemittel, betrieben werden kann. Das fünfte Trennprodukt verlässt die Trennung daher auf einem entsprechen niedrigen Temperaturniveau und kann auf diesem als Teil des ersten Trenneinsatzes erneut in die absorptive Trennung geführt werden. Ein Sumpfverdampfer der zur destillativen Trennung des dritten Trenneinsatzes verwendeten Destillationssäule kann beispielsweise mit Hochdruckpropylen beheizt werden. Der erste Trenneinsatz kann durch die Zuspeisung des fünften Trennprodukts etwas abgekühlt werden.

Als Absorptionsflüssigkeiten in der absorptiven Trennung des ersten Trenneinsatzes können im Rahmen der vorliegenden Erfindung beispielsweise Flüssigerdgas (engl. Liquefied Petroleum Gas, LPG) oder auch C4-, C5- oder schwerere Kohlenwasserstoffe und entsprechende Gemische verwendet werden. Besondere Vorteile bieten butanreiche Gemische, insbesondere sogenanntes C4-LPG. Die Absorptionsflüssigkeit kann grundsätzlich Kohlenwasserstoffe mit drei, vier, fünf, sechs, sieben, acht, neun und/oder zehn Kohlenstoffatomen und/oder beliebige Gemische in einer Menge von jeweils mehr als 10%, 20%, 30%, 40%, 50%, 60%, 70%, 80% oder 90% aufweisen, weist jedoch insbesondere überwiegend oder ausschließlich Kohlenwasserstoffe mit vier, fünf und sechs, vorzugsweise überwiegend oder ausschließlich Kohlenwasserstoffe mit vier und fünf Kohlenstoffatomen auf.

Wie bereits eingangs erläutert, ist es in herkömmlichen Verfahren zur Bearbeitung entsprechender Einsatzströme erforderlich, ausgesprochen tiefe Temperaturen von bis zu -100 °C und ggf. auch weniger bereitzustellen und ggf. zusätzlich Turboexpander einzusetzen. Beides schafft Nachteile, weil bei entsprechend tiefen Temperaturen teure, tieftemperaturfeste Materialien verwendet werden müssen bzw. besondere Anforderungen an drehende Maschinen zu stellen sind.

Die Erfindung behebt diesen Nachteil durch das Auswaschen von Kohlenwasserstoffen mit zwei Kohlenstoffatomen aus einem entsprechenden Strom und die nachgeschaltete Trennung. Sie ersetzt damit die klassischen Tieftemperaturkomponenten in herkömmlichen Anlagen. Der als Ausgangsgemisch verwendete C2minus-Strom wird dabei absorptiv von C2-Komponenten befreit, die mittels der mehrfach erwähnten Absorptionsflüssigkeit ausgewaschen werden.

Bei dem als Ausgangsgemisch verwendeten C2-Strom kann es sich beispielsweise um den Austrittsstrom einer C2-Hydrierung einer entsprechenden Ethylen- bzw. Dampfspaltanlage handeln. Ein entsprechender Einsatzstrom muss zur Verwendung im Rahmen der vorliegenden Erfindung, wie auch zuvor erläutert, vorteilhafterweise nicht weiter abgekühlt werden. Dies wird unter anderem deshalb ermöglicht, weil das (kalte) fünfte Trennprodukt ebenfalls bei der Bildung des ersten Trenneinsatzes verwendet wird.

Die Erfindung schafft den besonderen Vorteil, dass, insbesondere in Kombination mit einem angeschlossenen, bei vergleichsweise hohem Druck betriebenen C2-Splitter lediglich das durch C3-Kältemittel bereitstellbare Kälteniveau erforderlich ist. Auf einen Ethylenkältekreislauf kann daher verzichtet werden. Die Erfindung eignet sich in gleicher Weise für sämtliche Typen von Ethylenanlagen, und ist daher, im Gegensatz zu herkömmlichen Verfahren, unabhängig von den verwendeten Einsätzen wie Naphtha, Ethan oder Propan. Aufgrund der wärmeren Prozesstemperatur kann einerseits teures Material eingespart werden, andererseits ergibt sich aber auch eine besonders einfache Prozessführung.

Im Rahmen der vorliegenden Erfindung ist es besonders vorteilhaft, wenn die Absorptionsflüssigkeit unter Verwendung des vierten Trennprodukts gebildet wird. Dabei kommt die destillative Trennung des zweiten Trenneinsatzes einem "Regenerieren" einer entsprechenden Absorptionsflüssigkeit gleich. Zur Entfernung der Kohlenwasserstoffe mit zwei Kohlenstoffatomen sowie von Methan und Wasserstoff aus dem zweiten Trennprodukt bzw. dem zweiten Trenneinsatz im Rahmen der destillativen Trennung wird vorteilhafterweise eine Destillationssäule verwendet, deren Kopfprodukt partiell mit C3-Kälte kondensiert werden kann. Die dabei anfallende Flüssigkeit wird als Rücklauf auf eine entsprechende Destillationssäule zurückgeführt. Die dabei nicht kondensierte Gasphase, d.h. das nicht kondensierte Kopfprodukt vom Kopf dieser Destillationssäule, enthält Kohlenwasserstoffe mit zwei Kohlenstoffatomen sowie Methan und Wasserstoff und stellt das dritte Trennprodukt dar. Eine entsprechende Destillationssäule wird mit einem Sumpfverdampfer betrieben, in den Wärme beispielsweise durch Mitteldruckdampf eingeleitet werden kann.

Vorteilhafterweise wird im Rahmen der vorliegenden Erfindung das vierte Trennprodukt bzw. die unter Verwendung des vierten Trennprodukts gebildete Absorptionsflüssigkeit abgekühlt. Die Abkühlung erfolgt dabei typischerweise auf mehreren Stufen, wie nachfolgend erläutert.

Die letzte Abkühlstufe bzw. die letzten Abkühlstufen können dabei das Abkühlen mit einem C3-Kältemittel auf unterschiedlichen Temperaturniveaus umfassen. Diese Temperaturniveaus umfassen typischerweise ein erstes Temperaturniveau bei -15 bis -25 °C, insbesondere bei ca. -20 °C, und/oder ein zweites Temperaturniveau bei -35 bis -40 °C, insbesondere bei ca. -38 °C. Auf diese Weise kann die Absorptionsflüssigkeit bei günstigen Temperaturen in eine zum Auswaschen der Kohlenwasserstoffe mit zwei Kohlenstoffatomen aus dem ersten Trenneinsatz verwendete Trenneinheit eingespeist werden.

Es ist besonders vorteilhaft, wenn unter Verwendung des sechsten Trennprodukts ein vierter Trenneinsatz gebildet und einer weiteren destillativen Trennung zugeführt wird, in der ein überwiegend Ethan und ein überwiegend Ethylen enthaltendes Trennprodukt gebildet werden. Hierbei kann der vierte Trenneinsatz beispielsweise einem sogenannten C2-Splitter zugeführt werden, in dem in diesem enthaltenes Ethan von in diesem enthaltenem Ethylen getrennt wird. Bei der Abkühlung der Absorptionsflüssigkeit kann dabei ein entsprechender Ethan- und/oder Ethylenstrom zum Einsatz kommen. Das vierte Trennprodukt oder die unter Verwendung des vierten Trennprodukts gebildete Absorptionsflüssigkeit kann also vorteilhafterweise unter Verwendung des überwiegend Ethan und/oder unter Verwendung des überwiegend Ethylen enthaltenden Trennprodukts abgekühlt werden. Dies erlaubt eine günstige Vorkühlung, ehe die regenerierte Absorptionsflüssigkeit anschließend der Abkühlung auf die zuvor erläuterten ersten und zweiten Temperaturniveaus zugeführt wird. Die destillative Trennung des vierten Trenneinsatzes erfolgt dabei vorteilhafterweise in einem sogenannten Hochdruck-C2-Splitter bei 18 bis 20 bar. In einem entsprechenden Hochdruck-C2-Splitter reichen ebenfalls C3-Kältemittel zur Trennung aus, beispielsweise kann ein Kopfkondensator des Hochdruck-C2-Splitters mit Kältemittel bei ca. -38 °C gekühlt und der Sumpfverdampfer mit Hochdruckpropylen beheizt werden.

Vorteilhafterweise wird im Rahmen der vorliegenden Erfindung zum Auswaschen der Kohlenwasserstoffe mit zwei Kohlenstoffatomen aus dem Ausgangsgemisch, d.h. für die absorptive Trennung, eine absorptive erste Trenneinheit verwendet, auf die auch die Absorptionsflüssigkeit aufgegeben wird. Zur destillativen Trennung des dritten Trenneinsatzes kommt eine destillative zweite Trenneinheit zum Einsatz. Besondere Vorteile, insbesondere im Hinblick auf Isolations- und Raumerfordernisse, ergeben sich, wenn die erste Trenneinheit und die zweite Trenneinheit baulich integriert ausgeführt sind, wobei die erste Trenneinheit oberhalb der zweiten Trenneinheit in einer gemeinsamen Außenhülle angeordnet und von der ersten Trenneinheit durch einen Flüssigkeitssperrboden getrennt ist. In diesem Fall steigt das fünfte Trennprodukt aus der zweiten Trenneinheit in die erste Trenneinheit auf, ohne dass zusätzliche Leitungen erforderlich sind.

Vorteilhafterweise umfasst das erfindungsgemäße Verfahren ferner, dass unter Verwendung des ersten Trennprodukts ein überwiegend Methan und Wasserstoff enthaltender Strom gebildet wird. Hierzu wird das erste Trennprodukt vorteilhafterweise teilweise oder vollständig durch einen Wärmetauscher geführt und anschließend in einen Abscheidebehälter eingespeist. Aus diesem Abscheidebehälter abgezogene flüssige und gasförmige Ströme stellen die überwiegend Methan beziehungsweise überwiegend Wasserstoff enthaltenden Ströme dar. Der flüssige Strom kann entspannt und zusammen mit dem gasförmige Strom zur Kühlung des ersten Abstroms im zuvor verwendeten Wärmetauscher eingesetzt werden. Eine weitere Auftrennung des gasförmigen Stroms, beispielsweise mittels einer PSA-Anlage, ist möglich.

Eine erfindungsgemäße Anlage zur trenntechnischen Bearbeitung eines Ausgangsgemischs, das überwiegend Wasserstoff, Methan und Kohlenwasserstoffe mit zwei Kohlenstoffatomen enthält, umfasst Mittel, die zum Bilden eines ersten Trenneinsatzes unter Verwendung des Ausgangsgemischs und zum absorptiven Trennen des ersten Trenneinsatzes unter Verwendung einer Absorptionsflüssigkeit, die überwiegend Kohlenwasserstoffe mit zumindest drei Kohlenstoffatomen enthält, eingerichtet sind, wobei das absorptive Trennen des ersten Trenneinsatzes das Erzeugen eines ersten Trennprodukts, das überwiegend Wasserstoff und Methan aus dem ersten Trenneinsatz enthält, und eines zweiten Trennprodukts, das Wasserstoff, Methan und Kohlenwasserstoffe mit zwei Kohlenstoffatomen aus dem ersten Trenneinsatz und Kohlenwasserstoffe mit zumindest drei Kohlenstoffatomen aus der Absorptionsflüssigkeit enthält, umfasst.

Erfindungsgemäß sind Mittel vorgesehen, die zum Bilden eines zweiten Trenneinsatzes unter Verwendung des zweiten Trennprodukts und zum destillativen Trennen des zweiten Trenneinsatzes eingerichtet sind, wobei das destillative Trennen des zweiten Trenneinsatzes das Erzeugen eines dritten Trennprodukts, das überwiegend Methan, Wasserstoff und Kohlenwasserstoffe mit zwei Kohlenstoffatomen enthält, und das Erzeugen eines vierten Trennprodukts, das überwiegend Kohlenwasserstoffe mit zumindest drei Kohlenstoffatomen enthält, umfasst. Ferner sind erfindungsgemäß Mittel vorgesehen, die zum Bilden eines dritten Trenneinsatzes unter Verwendung des dritten Trennprodukts und zum destillativen Trennen des dritten Trenneinsatzes eingerichtet sind, wobei das destillative Trennen des dritten Trenneinsatzes das Erzeugen eines fünften Trennprodukts, das überwiegend Methan, Wasserstoff und Kohlenwasserstoffe mit zwei Kohlenstoffatomen enthält, und das Erzeugen eines sechsten Trennprodukts, das überwiegend Kohlenwasserstoffe mit zwei Kohlenstoffatomen enthält, umfasst. Ferner sind Mittel vorgesehen, die dafür eingerichtet sind, das fünfte Trennprodukt beim Bilden des ersten Trenneinsatzes zu verwenden, d.h. das fünfte Trennprodukt oder einen Teil hiervon insbesondere zu dem Ausgangsgemisch oder einem Teil hiervon zuzuspeisen.

Ausführungsformen der Erfindung werden nachfolgend unter Bezugnahme auf die beigefügten Zeichnungen gegenüber dem Stand der Technik näher erläutert.

### Kurze Beschreibung der Zeichnungen

Figur 1 zeigt ein nicht erfindungsgemäßes Verfahren in Form eines schematischen Prozessflussdiagramms.
Figur 2 zeigt ein nicht erfindungsgemäßes Verfahren in Form eines schematischen Prozessflussdiagramms.
Figur 3 zeigt ein nicht erfindungsgemäßes Verfahren in Form eines schematischen Prozessflussdiagramms.
Figur 4 zeigt ein Verfahren gemäß einer Ausführungsform der Erfindung in Form eines schematischen Prozessflussdiagramms.
Figur 5 zeigt ein Verfahren gemäß einer Ausführungsform der Erfindung in Form eines schematischen Prozessflussdiagramms.

### Ausführliche Beschreibung der Zeichnungen

In den Figuren tragen einander entsprechende Elemente entsprechende Bezugszeichen und werden der Übersichtlichkeit halber nicht wiederholt erläutert.

In Figur 1 ist ein nicht erfindungsgemäßes Verfahren zur trenntechnischen Bearbeitung eines überwiegend Wasserstoff, Methan und Kohlenwasserstoffen mit zwei Kohlenstoffatomen enthaltenden Ausgangsgemischs in Form eines schematischen Prozessflussdiagramms veranschaulicht. Das Verfahren wird insbesondere zur trenntechnischen Bearbeitung eines Ausgangsgemischs eingesetzt, das aus einem Rohgasstrom eines Dampfspaltverfahrens stammt, bei dem überwiegend oder ausschließlich flüssige Einsätze, beispielsweise Naphtha, bearbeitet werden, d.h. aus einem Rohgasstrom eines "Flüssigcrackers".

Ein derartiges Ausgangsgemisch oder ein Teil davon wird dabei in Form des Stroms A zunächst durch einen ersten Wärmetauscher 101 geführt und unter anderem unter Verwendung eines Kältemittelstroms B, bei welchem es sich beispielsweise um Ethylen auf einem Temperaturniveau von ca. -57 °C handelt, abgekühlt.

Der Strom A wird anschließend in einen ersten Abscheidebehälter 102 überführt, welchem ein flüssiger Strom C und ein gasförmiger Strom D entnommen werden. Der gasförmige Strom D wird durch einen zweiten Wärmetauscher 103 geführt und hier unter anderem mit einem Strom E, bei welchem es sich um Ethylen auf einem Temperaturniveau von ca. -80 °C handelt, weiter abgekühlt.

Der Strom D wird anschließend in einen weiteren Abscheidebehälter 104 überführt, dem ebenfalls ein flüssiger Strom, hier mit F bezeichnet, und ein gasförmiger Strom, hier mit G bezeichnet, entnommen werden. Der Strom G wird durch einen dritten Wärmetauscher 105 geführt und dort unter anderem mit einem Strom H, bei welchem es sich um Ethylen auf einem Temperaturniveau von ca. -100 °C handelt, abgekühlt.

Der Strom G wird anschließend in eine Absorptionskolonne 106 überführt, welche mit einem methanreichen flüssigen Rücklaufstrom I betrieben wird. Aus dem Sumpf der Absorptionskolonne 106 wird ein flüssiger Strom K abgezogen und in dem Wärmetauscher 105 erwärmt.

Ein gasförmiger Strom L vom Kopf der Absorptionskolonne 106 wird in einem Wärmetauscher 107 weiter abgekühlt und anschließend in einen weiteren Abscheidebehälter 108, den sogenannten Wasserstoffabscheider, überführt. Aus dem Abscheidebehälter 108 flüssig bzw. gasförmig abgezogene Ströme M und N werden in den Wärmetauschern 107, 105, 103 und 101 erwärmt und als methanreiche Fraktion (Strom M) bzw. wasserstoffreiche Fraktion (Strom N) bereitgestellt.

Die bereits erwähnten flüssigen Ströme C, F und K werden in eine Destillationssäule 109 überführt, wobei ihre Einspeisung je nach Zusammensetzung und Temperatur in unterschiedlichen Höhen erfolgt. Die Destillationssäule 109, die auch als Demethanizer bezeichnet wird, wird mit einem Sumpfverdampfer 110 unter Verwendung eines typischen C3-Kältemittels betrieben. Vom Kopf der Destillationssäule 109 wird ein gasförmiger Strom O abgezogen und einem insgesamt mit 111 bezeichneten Kopfkondensator zugeführt. Der Kopfkondensator 111 umfasst dabei einen Wärmetauscher 112, der mit Ethylen auf einem Temperaturniveau von ca. -100 °C als Kältemittel betrieben wird. In einem dem Wärmetauscher 112 nachgeschalteten Abscheidebehälter 113 wird ein flüssiger Strom P erhalten, welcher mittels einer Pumpe 114 zu einem Teil als Rücklauf auf die Destillationssäule 109 und zu einem Teil als Rücklauf auf die Absorptionskolonne 106 in Form des Stroms I aufgegeben wird. Ein nicht verflüssigter Anteil des Stroms O wird in Form des Stroms Q aus dem Abscheidebehälter 113 abgezogen und als methanreicher Strom mit dem Strom M vereinigt.

Durch den erläuterten Betrieb der Destillationssäule 109 kann aus deren Sumpf ein flüssiger Strom R abgezogen werden, der reich an Kohlenwasserstoffen mit zwei Kohlenstoffatomen ist. Dieser wird im dargestellten Beispiel im Wärmetauscher 101 erwärmt und anschließend beispielsweise einer Trennung zur Gewinnung vom Ethan und Ethylen in einem sogenannten C2-Splitter unterworfen.

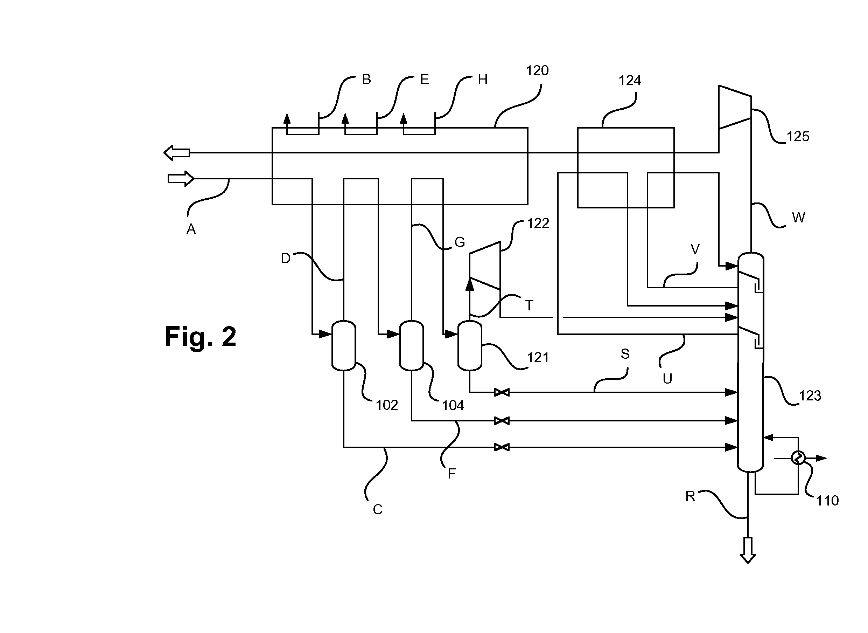

Während sich das in Figur 1 veranschaulichte Verfahren insbesondere für Ausgangsgemische eignet, die aus der Dampfspaltung von flüssigen Einsätzen, beispielsweise Naphtha, stammen, ist in Figur 2 ein Verfahren veranschaulicht, das sich insbesondere für die Bearbeitung von Ausgangsgemischen eignet, die aus einer Dampfspaltung von gasförmigen, insbesondere ethanreichen Einsätzen stammen, d.h. aus einem "Gascracker".

Das in Figur 2 veranschaulichte Verfahren umfasst teilweise ähnliche Verfahrensschritte bzw. Vorrichtungen, die daher in Figur 2 identisch wie in Figur 1 bezeichnet sind. Anstelle der drei getrennten Wärmetauscher 101, 103 und 105 der Figur 1 ist hier ein gemeinsamer Wärmetauscherblock 120 veranschaulicht, der jedoch ebenfalls mit entsprechenden Strömen wie in dem in Figur 1 gezeigten Verfahren, d.h. mit Ethylen auf einem Temperaturniveau von ca. -57 °C (Strom B), -80 °C (Strom E) und -100°C (Strom H) gekühlt wird. Auch die Gewinnung der flüssigen Ströme C und F in entsprechenden Abscheidebehältern 102 und 104 verläuft grundsätzlich ähnlich. Betont sei jedoch, dass in dem Verfahren, das in Figur 2 veranschaulicht ist, grundsätzlich andere Drücke zur Trennung zum Einsatz kommen können.

Anstelle der Absorptionskolonne 106, die in Figur 1 veranschaulicht ist, kommt hier jedoch ein weiterer Abscheidebehälter 121 zum Einsatz, in den der gasförmige Strom G eingespeist wird. Aus dem Abscheidebehälter 121 wird ein flüssiger Strom S und ein gasförmiger Strom T entnommen. Der Strom T wird in einem Turboexpander 122 entspannt und anschließend in eine, hier unterschiedlich zur Destillationssäule 109 des in der Fig. 1 dargestellten Verfahrens , insbesondere mit drei Abschnitten, ausgebildete und daher abweichend bezeichnete Destillationssäule 123 eingespeist. In die Destillationssäule 123, die einen vergleichbar mit dem Sumpfverdampfer 110 der Destillationssäule 109 gemäß Figur 1 aufgebauten und betriebenen Sumpfverdampfer 110 aufweist, werden ferner die zuvor erläuterten flüssigen Ströme C, F und S eingespeist.

Aus einem oberen Teil eines unteren Bereichs der Destillationssäule 123 wird ein gasförmiger Strom U abgezogen, in einem Wärmetauscher 124 abgekühlt und auf einen Mittelteil der Destillationssäule 123 aufgegeben. Aus einem oberen Bereich des Mittelteils der Destillationssäule 123 wird ein weiterer gasförmiger Strom V abgezogen, ebenfalls in dem Wärmetauscher 124 abgekühlt und in einen oberen Teil der Destillationssäule 123 eingespeist. Vom Kopf der Destillationssäule 123 bzw. deren oberen Teils wird ein Strom W gasförmig abgezogen und durch einen Turboexpander 125 entspannt. Die Entspannung des Stroms W in dem Turboexpander 125 liefert die zur Abkühlung der Ströme U und V erforderliche Kälte. Der Strom W trägt ferner zur Abkühlung in dem Wärmetauscher 120 bei. Aus dem Sumpf der Destillationssäule 123 wird schließlich ein Strom R mit vergleichbarer Zusammensetzung wie jener des Stroms R gemäß Figur 1 erhalten und beispielsweise einem C2-Splitter zugeführt.

Wie aus der Zusammenschau der Figuren 1 und 2 ersichtlich, ist in den hier gezeigten Verfahren die Verwendung von Ethylen als Kältemittel erforderlich, gemäß dem Verfahren, das wie in der Fig. 2 dargestellt ist, zusätzlich die Verwendung von zwei Turboexpandern.

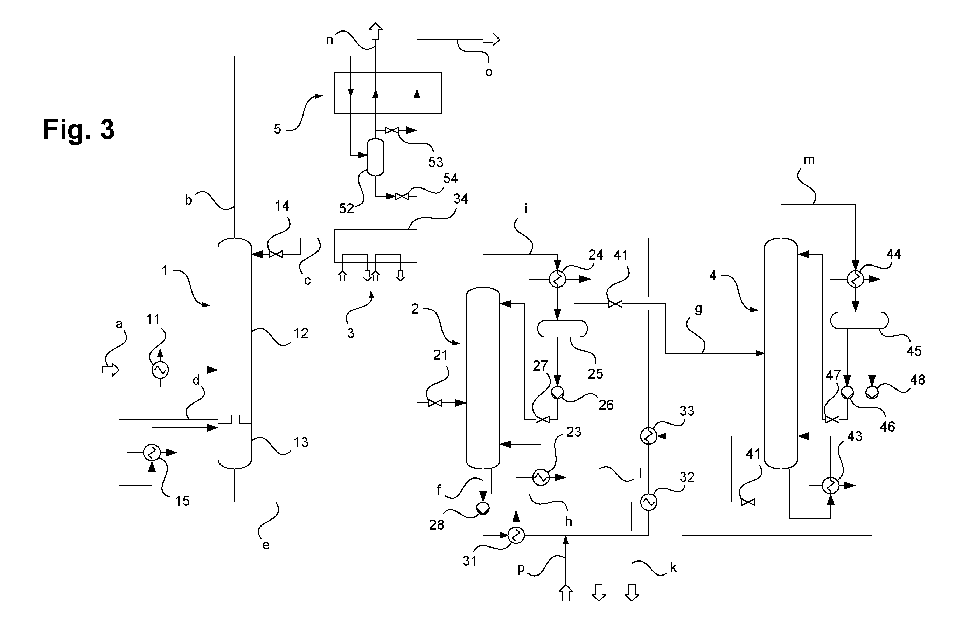

In Figur 3 ist ein weiteres nicht erfindungsgemäßes Verfahren zur trenntechnischen Bearbeitung eines an Wasserstoff, Methan und Kohlenwasserstoffen mit zwei Kohlenstoffatomen reichen Einsatzstroms in Form eines vereinfachten Prozessflussdiagramms veranschaulicht, das einen Verzicht auf entsprechend tiefe Temperaturen ermöglicht.

In dem in Figur 3 gezeigten Verfahren wird ein entsprechendes Ausgangsgemisch in Form des Stroms a, bzw. ein hieraus gebildeter Trenneinsatz (hier als "erster" Trenneinsatz bezeichnet), zunächst durch einen Wärmetauscher 11 geführt und anschließend in den oberen Teil 12 einer Absorptionskolonne 1 überführt. Der Wärmetauscher 11 kann beispielsweise mit einem Kältemittel bei -38 °C betrieben werden und kühlt den Strom a auf ca. -35 °C ab. Ein unterer Teil der Absorptionskolonne 1 ist mit 13 bezeichnet. Am Kopf des oberen Teils 12 der Absorptionskolonne 1 wird, wie nachfolgend erläutert, ein überwiegend Methan und Wasserstoff enthaltendes Trennprodukt (hier als "erstes" Trennprodukt bezeichnet) in Form des Stroms b abgezogen, dessen weitere Behandlung unten erläutert wird.

In dem oberen Teil 12 der Absorptionskolonne 1 werden die Kohlenwasserstoffe mit zwei Kohlenstoffatomen aus dem ersten Trenneinsatz bzw. dem Strom a durch eine Absorptionsflüssigkeit in Form des Stroms c überwiegend oder vollständig ausgewaschen, wobei die Absorptionsflüssigkeit im dargestellten Beispiel über ein Ventil 14 auf den oberen Teil 12 der Absorptionskolonne 1 aufgegeben wird.

Im Sumpf des oberen Teils 12 der Absorptionskolonne 1 bzw. auf einem Flüssigkeitssperrboden scheidet sich eine Flüssigkeit ab, die neben den Kohlenwasserstoffen mit zwei Kohlenstoffatomen aus dem ersten Trenneinsatz bzw. dem Strom a und den höheren Kohlenwasserstoffen der Absorptionsflüssigkeit des Stroms c auch noch Methan enthält. Zum Entfernen des Methans wird diese Flüssigkeit daher im dargestellten Beispiel in Form des Stroms d abgezogen, durch einen Aufkocher 15 geführt, der beispielsweise mit Waschwasser betrieben wird, und in den unteren Teil 13 der Absorptionskolonne 1 eingespeist. Auf diese Weise kann Methan zumindest teilweise aus dem Fluid des Stroms d desorbiert werden, so dass sich im Sumpf der Absorptionskolonne 1 bzw. deren unteren Teils 13 eine Sumpfflüssigkeit abscheidet, die reich an Kohlenwasserstoffen mit zwei Kohlenstoffatomen, jedoch arm an leichteren Kohlenwasserstoffen und Wasserstoff ist.

Die Sumpfflüssigkeit wird im dargestellten Beispiel in Form eines Abstroms e gewonnen und über ein Ventil 21 in eine Destillationssäule 2 überführt. Die Destillationssäule 2 dient zur Auftrennung des Abstroms e und zur Gewinnung eines an Kohlenwasserstoffen mit zumindest drei Kohlenstoffatomen reichen Stroms f und eines an Kohlenwasserstoffen mit zwei Kohlenstoffatomen reichen Stroms g. Zur Gewinnung der genannten Ströme f und g wird aus dem Sumpf der Destillationssäule 2 ein Strom h abgezogen, in einem Wärmetauscher 23, der einen Sumpfverdampfer der Destillationssäule 2 bildet, verdampft, und zurück in die Destillationssäule 2 geführt. Der Wärmetauscher 23 kann mit Niederdruckdampf als Heizmedium betrieben werden. Zudem wird der bereits erwähnte, überwiegend Kohlenwasserstoffe mit zumindest drei Kohlenstoffatomen enthaltende Strom f aus der Destillationssäule 2 abgezogen und wie nachfolgend erläutert weiter behandelt.

Vom Kopf der Destillationssäule 2 wird ein gasförmiger Strom i abgezogen und durch einen Wärmetauscher 24 abgekühlt. Der Wärmetauscher 24 kann beispielsweise mit Propylen als Kältemittel auf -38 °C betrieben werden. Anschließend wird der Strom i in einen Abscheidebehälter 25 überführt. Vom Sumpf des Abscheidebehälters 25 wird ein nicht näher bezeichneter Strom abgezogen und mittels einer Pumpe 26 über ein Ventil 27 auf den Kopf der Destillationssäule 2 zurückgeführt.

Ein in dem Abscheidebehälter 25 gasförmig verbleibender Teil wird über ein Ventil 41 in eine weitere Destillationssäule 4 als der bereits erwähnte Strom g überführt. Die weitere Destillationssäule 4 dient zur Auftrennung von in dem Strom g, also dem dritten Abstrom, enthaltenen Kohlenwasserstoffen mit zwei Kohlenstoffatomen, insbesondere Ethan und Ethylen, wobei ein ethylenreicher Strom in Form des Stroms k und ein ethanreicher Strom in Form des Stroms I bereitgestellt wird. Zur Bereitstellung des Stroms k wird vom Kopf der Destillationssäule 4 ein Strom m abgezogen und durch einen Wärmetauscher 44, der beispielsweise ebenfalls mit Propylen- bzw. C3-Kälte bei -38 °C betrieben wird, gekühlt. Fluid des Stroms m wird anschließend in einen Abscheidebehälter 45 überführt, von dessen Sumpf einerseits ein nicht näher bezeichneter Strom abgezogen und mittels einer Pumpe 46 und eines Ventils 47 zum Kopf der Destillationssäule 4 zurückgeführt wird. Ein weiterer Teil wird in Form des Stroms k abgezogen und mittels einer Pumpe 48 über einen Wärmetauscher 32 aus der Anlage ausgeführt.

Zur Bereitstellung des Stroms I wird aus dem Sumpf der Destillationssäule 4, die ferner mit einem Sumpfverdampfer 43 betrieben wird, der Strom I über ein Ventil 41 ausgeleitet und über einen Wärmetauscher 33 aus der Anlage herausgeführt. Der Wärmetauscher 43 des Sumpfverdampfers der Destillationssäule 4 wird beispielsweise mittels Hochdruckpropylen als Wärmemedium betrieben. Der bereits oben erwähnte Strom f, also ein Strom, der aus dem zweiten Abstrom e gebildet wird und überwiegend oder ausschließlich Kohlenwasserstoffe mit zumindest drei Kohlenstoffatomen enthält, wird aus der Destillationssäule 2 mittels einer Pumpe 28 durch einen Wärmetauscher 31, der beispielsweise mittels Kühlwasser betrieben wird, und anschließend durch die bereits erwähnten Wärmetauscher 32 und 33 geführt. Zuvor wird der Strom f mit einem frischen Absorptionsflüssigkeitsstrom p, der reich an Kohlenwasserstoffen mit zumindest drei Kohlenstoffatomen ist, vereinigt.

Nach der Vereinigung der Ströme f und p und der Abkühlung in den genannten Wärmetauschern 32 und 33 wird ein entsprechend vereinigter Strom c, die bereits erläuterte Absorptionsflüssigkeit, durch einen weiteren Wärmetauscher 34, beispielsweise einen Plattenwärmetauscher, der mit zwei getrennten Propylenkältemittelströmen auf unterschiedlichen Temperaturniveaus, beispielsweise - 38 °C und -20 °C betrieben wird, geführt. Der auf diese Weise auf eine Temperatur von ca. -35 °C abgekühlte Strom c wird über das erläuterte Ventil 14 auf die Absorptionskolonne 1 aufgegeben.

Vom Kopf der Absorptionskolonne 1 bzw. deren oberen Teils wird der bereits erwähnte erste Abstrom b abgezogen und zur Gewinnung eines wasserstoffreichen Stroms n und eines methanreichen Stroms o wie nachfolgend erläutert behandelt.

Der erste Abstrom b wird zunächst durch einen Wärmetauscher 5 geführt und in einen Abscheidebehälter 52 eingespeist. Am Kopf des Abscheidebehälters 52 wird eine wasserstoffreiche Fraktion in Form des erläuterten Stroms n abgezogen, aus dem Sumpf des Abscheidebehälters 52 wird eine überwiegend Methan enthaltende Fraktion über ein Ventil 54 abgezogen. Der Strom o kann durch eine Vereinigung mit zumindest einem Teil des Stroms n bereitgestellt werden, wozu ein Ventil 53 vorgesehen ist. Damit kann eine gewünschte Reinheit des Stroms n eingestellt werden.

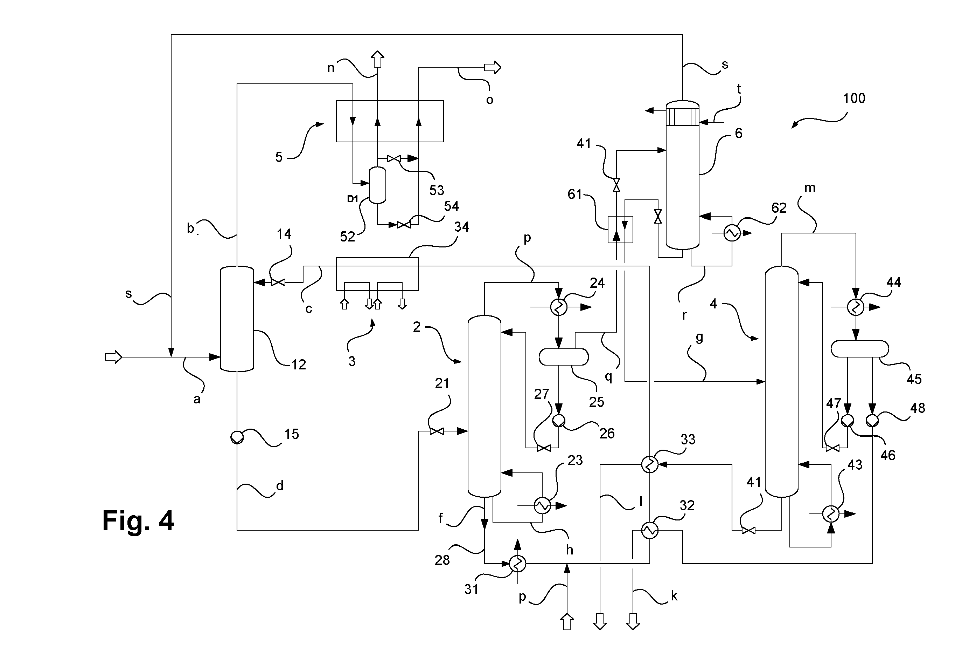

In Figur 4 ist ein Verfahren zur trenntechnischen Bearbeitung eines an Wasserstoff, Methan und Kohlenwasserstoffen mit zwei Kohlenstoffatomen reichen Einsatzstroms gemäß einer Ausführungsform der Erfindung in Form eines vereinfachten Prozessflussdiagramms veranschaulicht und insgesamt mit 100 bezeichnet. Das Verfahren 100 ermöglicht wie das in Figur 3 gezeigte Verfahren einen Verzicht auf tiefe Temperaturen (Ethylenkälte) und besitzt weitere energetische Vorteile. Verfahrensschritte bzw. Vorrichtungen, die den in Figur 3 gezeigten Verfahrensschritten bzw. Vorrichtungen entsprechen, sind identisch bezeichnet. Entsprechendes gilt für Ströme vergleichbarer Zusammensetzung (aber nicht notwendigerweise vergleichbarer Temperatur und vergleichbaren Drucks).

In dem in Figur 4 gezeigten Verfahren 100 wird der Strom a nicht wie gemäß Figur 3 durch einen Wärmetauscher geführt, sondern direkt (zusammen mit einem unten erläuterten Strom s) als "erster Trenneinsatz" in eine einteilig ausgebildete Absorptionskolonne eingespeist, die hier (wie der obere Teil der Absorptionskolonne 1 gemäß Figur 3, die dieser grundsätzlich funktional entspricht) mit 12 bezeichnet ist. Der Strom a ist, wie zuvor erläutert, reich an Wasserstoff, Methan und Kohlenwasserstoffen mit zwei Kohlenstoffatomen und enthält ggf. Spuren an Kohlenwasserstoffen mit drei Kohlenstoffatomen. Seine Temperatur beim Eintritt in die Absorptionskolonne 12 beträgt beispielsweise ca. -32 °C.

Am Kopf der Absorptionskolonne 12 wird auch hier ein aus Fluid des Einsatzstroms a gebildeter, an Methan und Wasserstoff reicher und Kohlenwasserstoffen mit zwei Kohlenstoffatomen armer Abstrom b abgezogen ("erstes Trennprodukt"), dessen weitere Behandlung bereits bezüglich Figur 3 erläutert wurde. Auf die obigen Erläuterungen sei daher an dieser Stelle ausdrücklich verwiesen.

In der Absorptionskolonne 12 werden die Kohlenwasserstoffe mit zwei Kohlenstoffatomen, ähnlich wie gemäß Figur 3, aus dem Fluid des Einsatzstroms a durch Inkontaktbringen mit einer an Kohlenwasserstoffen mit zumindest drei Kohlenstoffatomen reichen Absorptionsflüssigkeit in Form des Stroms c überwiegend oder vollständig ausgewaschen, wobei die Absorptionsflüssigkeit auch hier über ein Ventil 14 auf die Absorptionskolonne 12 aufgegeben wird.

Im Sumpf der Absorptionskolonne 12 scheidet sich eine Flüssigkeit ab, die wie die Flüssigkeit auf dem Flüssigkeitssperrboden des oberen Teils 12 der Absorptionskolonne 1 gemäß Figur 3 neben den Kohlenwasserstoffen mit zwei Kohlenstoffatomen aus dem Fluid des Einsatzstroms a und den höheren Kohlenwasserstoffen der Absorptionsflüssigkeit c auch noch Methan und Wasserstoff enthält. Das gelöste Methan und Wasserstoff wird gemäß dem in Figur 4 gezeigten Verfahren 100 jedoch zunächst nicht entfernt, so dass ein gemäß Figur 4 aus dem Sumpf der Absorptionskolonne 12 abgezogener Strom d ("zweites Trennprodukt") noch Methan und Wasserstoff enthält.

Der Strom d wird im dargestellten Beispiel über ein Ventil 21 (als "zweiter Trenneinsatz") in eine Destillationssäule 2 überführt, die ähnlich wie die Destillationssäule 2 der Figur 3, jedoch auf einem höheren Druckniveau als die Absorptionskolonne 1, betrieben wird. Die Absorptionskolonne 1 wird beispielsweise auf einem Druckniveau von ca. 30 bar betrieben, die Destillationssäule 2 beispielsweise auf einem Druckniveau von ca. 33 bar. Der Strom d wird dabei mittels einer Pumpe 15 auf Druck gebracht.

Da der Strom d (also der "zweite Trenneinsatz") noch Methan und Wasserstoff enthält, geht dies zusammen mit den Kohlenwasserstoffen mit zwei Kohlenstoffatomen in den Kopfstrom der Destillationssäule 2 über, der hier mit p bezeichnet ist. Im Gegensatz zu dem in Figur 3 veranschaulichten Verfahren wird der Strom p in dem Wärmetauscher 24 beispielsweise mit Mitteldruckpropylen als Kältemittel auf nur-20 °C statt-38 °C gekühlt, was eine weitere Verringerung des Energieverbrauchs zur Folge hat. Ansonsten ist der Betrieb des Abscheidebehälters 25, der Pumpe 26 und des Ventils 27 in dem Verfahren 100 gemäß Figur 4 vergleichbar zu dem in Figur 3 veranschaulichten Verfahren.

Ein nicht verflüssigter Anteil des Stroms p ("drittes Trennprodukt") wird in Form des Stroms q zunächst in einem Gegenstromwärmetauscher 61 abgekühlt und anschließend über ein auch hier mit 41 bezeichnetes Ventil (als "dritter Trenneinsatz") in eine als Demethanizer betriebene Destillationssäule 6 überführt. Der Strom h wird in dem Verfahren 100 gemäß Figur 4 in dem Wärmetauscher 23 auf eine höhere Temperatur als gemäß Figur 3 angewärmt, wobei beispielsweise Mitteldruckdampf eingesetzt werden kann. Auch hier kann ein überwiegend Kohlenwasserstoffe mit zumindest drei Kohlenstoffatomen enthaltender Strom f ("viertes Trennprodukt") gewonnen werden. Die weitere Behandlung des Stroms f entspricht weitgehend jener gemäß Figur 3, so dass auf die Erläuterungen dort verwiesen werden kann.

Ein Sumpfstrom r der Destillationssäule 6 wird in einem Wärmetauscher 62 beispielsweise mit Hochdruckpropylen verdampft, der Kopf der Destillationssäule 6 wird mittels Propylenstroms t auf beispielsweise ca. -35 °C gekühlt. Aus dem Sumpf der Destillationssäule 6 kann daher ein weitgehend oder vollständig von Methan befreiter Strom g ("sechstes Trennprodukt"), der damit überwiegend Kohlenwasserstoffe mit zwei Kohlenstoffatomen enthält, abgezogen werden. Der Strom g wird durch den Gegenstromwärmetauscher 61 geführt und erwärmt. Die weitere Behandlung dieses Stroms g in dem Verfahren 100 gemäß Figur 4 erfolgt wie bereits zu dem Verfahren gemäß Figur 3 erläutert.

Vom Kopf der Destillationssäule 6 kann ein Strom s ("fünftes Trennprodukt"), der (nahezu) sämtliches Methan und Wasserstoff des Stroms q, aber auch Anteile von Kohlenwasserstoffen mit zwei Kohlenstoffatomen enthält, abgezogen werden. Der Strom s kann mit dem Strom a vereinigt bzw. bei der Bildung desselben verwendet werden. Auf diese Weise können die Kohlenwasserstoffe mit zwei Kohlenstoffatomen zurückgewonnen werden. Die Destillationssäule 6 und die Absorptionskolonne 12 können auf demselben Druckniveau betrieben werden, wobei ein leichtes Druckgefälle eine Überführung des Stroms s in die Absorptionskolonne 12 ohne Verdichter sicherstellen kann.

Das Verfahren 100 gemäß Figur 4 ist energetisch vorteilhaft gegenüber dem Verfahren, das in Figur 3 veranschaulicht ist, weil eine nochmals deutlich geringere Kälteleistung erforderlich ist. Wie erwähnt, kann der Wärmetauscher 24 mit einem Kältemittel bei -20 °C betrieben werden und auf eine Vorkühlung des Stroms a (siehe Wärmetauscher 11 gemäß Figur 3) kann verzichtet werden. Die als Demethanizer betriebene Destillationssäule 6 kann mit Kältemittel bei -38 °C betrieben werden, weil hier nur eine unscharfe Trennung möglich ist (Kohlenwasserstoffe mit zwei Kohlenstoffatomen können teilweise in den Strom s übergehen und können durch dessen Rückführung zurückgewonnen werden). Durch die Verwendung des Gegenstromwärmetauschers 61 kann ferner Kälte von dem Strom g auf den Strom q übertragen werden.

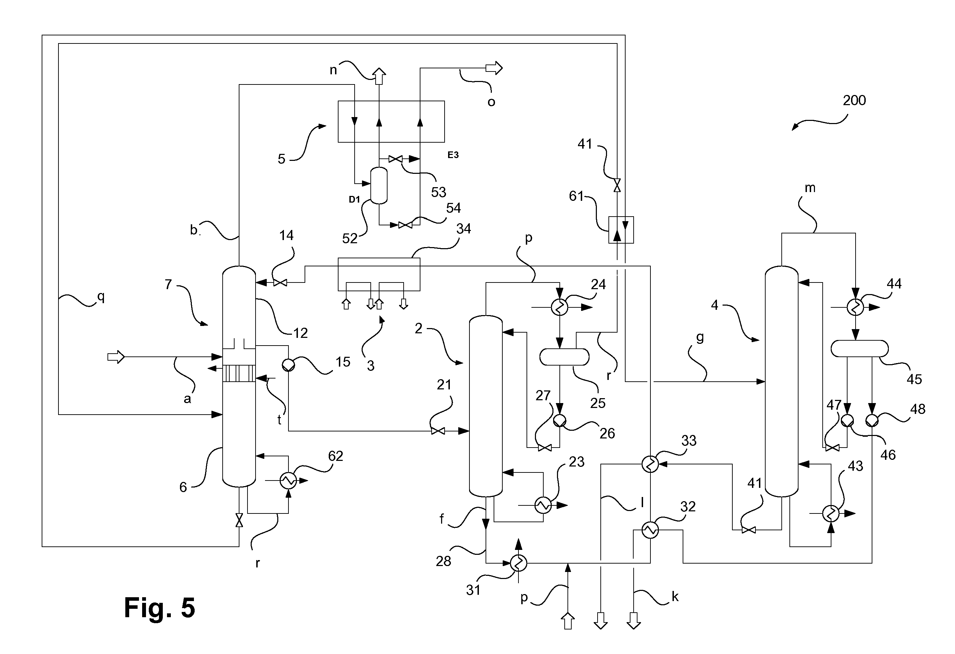

Eine Alternative des in Figur 4 veranschaulichten Verfahrens ist als weitere Ausführungsform der Erfindung in Figur 5 gezeigt und insgesamt mit 200 bezeichnet. Im Gegensatz zu dem in Figur 4 veranschaulichten Verfahren 100 sind hier die Absorptionskolonne 12 und die Destillationssäule 6 als baulich integriert als Einheit 7 ausgebildet, d.h. als Teile einer gemeinsamen Säule, die mittels eines Flüssigkeitssperrbodens bzw. Kaminhalsbodens voneinander getrennt sind. Dies ermöglicht einerseits einen Verzicht auf eine Leitung zur Überführung des Stroms s in die Säule 12 bzw. zur Zuspeisung des Stroms s zu dem Strom a. Ein dem Strom s entsprechendes Fluid (also das "fünfte Trennprodukt") steigt hier von der Destillationssäule 6 in die Absorptionskolonne 12 auf. Die Absorptionskolonne 12 und die Destillationssäule 6 können als integrierte Einheit 7 mittels einer gemeinsamen Kälteisolierung versehen werden, so dass sich der bauliche Aufwand verringert.

Zu weiteren Merkmalen und Vorteilen des Verfahrens 200 gemäß Figur 5 sei auf die Erläuterungen zu dem Verfahren 100 gemäß Figur 4 verwiesen.

## Patentansprüche

1. Verfahren (100, 200) zur trenntechnischen Bearbeitung eines Ausgangsgemischs, das überwiegend Wasserstoff, Methan und Kohlenwasserstoffe mit zwei Kohlenstoffatomen enthält, wobei das Verfahren (100, 200) umfasst:
- Bilden eines ersten Trenneinsatzes unter Verwendung des Ausgangsgemischs und absorptives Trennen des ersten Trenneinsatzes unter Verwendung einer Absorptionsflüssigkeit, die überwiegend Kohlenwasserstoffe mit zumindest drei Kohlenstoffatomen enthält, wobei
- das absorptive Trennen des ersten Trenneinsatzes das Erzeugen eines ersten Trennprodukts, das überwiegend Wasserstoff und Methan aus dem ersten Trenneinsatz enthält, und eines zweiten Trennprodukts, das Wasserstoff, Methan und Kohlenwasserstoffe mit zwei Kohlenstoffatomen aus dem ersten Trenneinsatz und Kohlenwasserstoffe mit zumindest drei Kohlenstoffatomen aus der Absorptionsflüssigkeit enthält, umfasst,
**gekennzeichnet durch**
- Bilden eines zweiten Trenneinsatzes unter Verwendung des zweiten Trennprodukts und destillatives Trennen des zweiten Trenneinsatzes, wobei das destillative Trennen des zweiten Trenneinsatzes das Erzeugen eines dritten Trennprodukts, das überwiegend Methan, Wasserstoff und Kohlenwasserstoffe mit zwei Kohlenstoffatomen enthält, und das Erzeugen eines vierten Trennprodukts, das überwiegend Kohlenwasserstoffe mit zumindest drei Kohlenstoffatomen enthält, umfasst,
- Bilden eines dritten Trenneinsatzes unter Verwendung des dritten Trennprodukts und destillatives Trennen des dritten Trenneinsatzes, wobei das destillative Trennen des dritten Trenneinsatzes das Erzeugen eines fünften Trennprodukts, das überwiegend Methan, Wasserstoff und Kohlenwasserstoffe mit zwei Kohlenstoffatomen enthält, und das Erzeugen eines sechsten Trennprodukts, das überwiegend Kohlenwasserstoffe mit zwei Kohlenstoffatomen enthält, umfasst, und
- Verwenden des fünften Trennprodukts beim Bilden des ersten Trenneinsatzes.

2. Verfahren nach Anspruch 1, bei dem das absorptive Trennen des ersten Trenneinsatzes auf einem ersten Druckniveau und das destillative Trennen des zweiten Trenneinsatzes auf einem zweiten Druckniveau durchgeführt wird, wobei das zweite Druckniveau 1 bis 10 bar oberhalb des ersten Druckniveaus liegt.

3. Verfahren nach Anspruch 2, bei dem das zweite Trennprodukt oder der unter Verwendung des zweiten Trennprodukts gebildete zweite Trenneinsatz in flüssiger Form unter Verwendung einer Pumpe auf das zweite Druckniveau oder ein Druckniveau oberhalb des zweiten Druckniveaus gebracht wird.

4. Verfahren nach Anspruch 1 oder 2, bei dem das destillative Trennen des dritten Trenneinsatzes auf dem ersten Druckniveau durchgeführt wird.

5. Verfahren nach einem der vorstehenden Ansprüche, bei dem das absorptive Trennen auf einem Temperaturniveau von -40 bis -27 °C durchgeführt wird.

6. Verfahren nach einem der vorstehenden Ansprüche, bei dem das dritte Trennprodukt bei dem destillativen Trennen des zweiten Trenneinsatzes auf einem Temperaturniveau von -25 bis -10 °C bereitgestellt wird.

7. Verfahren nach einem der vorstehenden Ansprüche, bei dem das fünfte Trennprodukt bei dem destillativen Trennen des dritten Trenneinsatzes auf einem Temperaturniveau von -40 bis -30 °C bereitgestellt wird.

8. Verfahren nach einem der vorstehenden Ansprüche, bei dem die bei der absorptiven Trennung des ersten Trenneinsatzes verwendete Absorptionsflüssigkeit unter Verwendung des vierten Trennprodukts gebildet wird.

9. Verfahren nach Anspruch 8, bei das vierte Trennprodukt oder die unter Verwendung des vierten Trennprodukts gebildete Absorptionsflüssigkeit auf ein Temperaturniveau von -40 bis -30 °C abgekühlt wird.

10. Verfahren nach einem der vorstehenden Ansprüche, bei dem unter Verwendung des sechsten Trennprodukts ein vierter Trenneinsatz gebildet und einer destillativen Trennung unterworfen wird, in der ein überwiegend Ethan und ein überwiegend Ethylen enthaltendes Trennprodukt gebildet werden.

11. Verfahren nach Anspruch 10, bei dem das vierte Trennprodukt oder die unter Verwendung des vierten Trennprodukts gebildete Absorptionsflüssigkeit unter Verwendung des überwiegend Ethan und/oder unter Verwendung des überwiegend Ethylen enthaltenden Trennprodukts abgekühlt wird.

12. Verfahren nach einem der vorstehenden Ansprüche, bei dem die absorptive Trennung des ersten Trenneinsatzes in einer ersten Trenneinheit (12) durchgeführt wird und bei dem die destillative Trennung des dritten Trenneinsatzes in einer zweiten Trenneinheit (6) durchgeführt wird.

13. Verfahren nach Anspruch 12, bei dem die erste Trenneinheit (12) und die zweite Trenneinheit (6) als integrierte bauliche Einheit (7) ausgeführt sind, wobei die erste Trenneinheit (12) oberhalb der zweiten Trenneinheit (6) angeordnet und von dieser durch einen Flüssigkeitssperrboden getrennt ist.

14. Verfahren nach einem der vorstehenden Ansprüche, bei dem aus Fluid des ersten Trennprodukts ein überwiegend Methan enthaltender Strom und ein überwiegend Wasserstoff enthaltender Strom gebildet werden.

15. Anlage (100, 200) zur trenntechnischen Bearbeitung eines Ausgangsgemischs, das überwiegend Wasserstoff, Methan und Kohlenwasserstoffe mit zwei Kohlenstoffatomen enthält, wobei die Anlage (100, 200) aufweist:
- Mittel (12), die zum Bilden eines ersten Trenneinsatzes unter Verwendung des Ausgangsgemischs und zum absorptiven Trennen des ersten Trenneinsatzes unter Verwendung einer Absorptionsflüssigkeit, die überwiegend Kohlenwasserstoffe mit zumindest drei Kohlenstoffatomen enthält, eingerichtet sind, wobei
- das absorptive Trennen des ersten Trenneinsatzes das Erzeugen eines ersten Trennprodukts, das überwiegend Wasserstoff und Methan aus dem ersten Trenneinsatz enthält, und eines zweiten Trennprodukts, das Wasserstoff, Methan und Kohlenwasserstoffe mit zwei Kohlenstoffatomen aus dem ersten Trenneinsatz und Kohlenwasserstoffe mit zumindest drei Kohlenstoffatomen aus der Absorptionsflüssigkeit enthält, umfasst
**gekennzeichnet durch**
- Mittel (2), die zum Bilden eines zweiten Trenneinsatzes unter Verwendung des zweiten Trennprodukts und zum destillativen Trennen des zweiten Trenneinsatzes eingerichtet sind, wobei das destillative Trennen des zweiten Trenneinsatzes das Erzeugen eines dritten Trennprodukts, das überwiegend Methan, Wasserstoff und Kohlenwasserstoffe mit zwei Kohlenstoffatomen enthält, und das Erzeugen eines vierten Trennprodukts, das überwiegend Kohlenwasserstoffe mit zumindest drei Kohlenstoffatomen enthält, umfasst,
- Mittel (6), die zum Bilden eines dritten Trenneinsatzes unter Verwendung des dritten Trennprodukts und zum destillativen Trennen des dritten Trenneinsatzes eingerichtet sind, wobei das destillative Trennen des dritten Trenneinsatzes das Erzeugen eines fünften Trennprodukts, das überwiegend Methan, Wasserstoff und Kohlenwasserstoffe mit zwei Kohlenstoffatomen enthält, und das Erzeugen eines sechsten Trennprodukts, das überwiegend Kohlenwasserstoffe mit zwei Kohlenstoffatomen enthält, umfasst, und
- Mittel, die dafür eingerichtet sind, das fünfte Trennprodukt beim Bilden des ersten Trenneinsatzes zu verwenden.
